(19) Europäisches Patentamt
European Patent Office
Office européen des brevets

(11) **EP 4 561 305 B1**

(12) **EUROPEAN PATENT SPECIFICATION**

(45) Date of publication and mention
of the grant of the patent:
**15.04.2026 Bulletin 2026/16**

(21) Application number: **24196428.7**

(22) Date of filing: **26.08.2024**

(51) International Patent Classification (IPC):
$H10H\ 20/855^{(2025.01)}$   $G02F\ 1/21^{(2006.01)}$
$H10H\ 29/855^{(2025.01)}$   $G02B\ 1/00^{(2006.01)}$

(52) Cooperative Patent Classification (CPC):
**H10H 20/855; G02B 1/002; G02F 1/29;
H10H 29/855;** G02F 2202/30

(54) **INTEGRATED OPTICAL ELEMENT AND FORMING METHOD OF METASURFACE**

INTEGRIERTES OPTISCHES ELEMENT UND VERFAHREN ZUR HERSTELLUNG EINER METAOBERFLÄCHE

ÉLÉMENT OPTIQUE INTÉGRÉ ET PROCÉDÉ DE FORMATION DE MÉTASURFACE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **21.11.2023 TW 112145016**

(43) Date of publication of application:
**28.05.2025 Bulletin 2025/22**

(73) Proprietor: **HON HAI PRECISION INDUSTRIES
CO., LTD.
New Taipei City 236038 (TW)**

(72) Inventors:
• **HONG, Yu-Heng**
**236038 New Taipei City (TW)**
• **HSU, Wen-Cheng**
**236038 New Taipei City (TW)**
• **MIAO, Wen-Chien**
**236038 New Taipei City (TW)**
• **HUANG, Yao-Wei**
**236038 New Taipei City (TW)**
• **KUO, Hao-Chung**
**236038 New Taipei City (TW)**

(74) Representative: **Winter, Brandl - Partnerschaft
mbB
Alois-Steinecker-Straße 22
85354 Freising (DE)**

(56) References cited:
**CN-A- 113 851 573     US-A1- 2019 383 982
US-A1- 2024 178 354**

• **ZHANG JINQIANNAN ET AL: "Gate-tunable
optical filter based on conducting oxide
metasurface heterostructure", vol. 44, no. 15, 18
July 2019 (2019-07-18), US, pages 3653,
XP093248896, ISSN: 0146-9592, Retrieved from
the Internet <URL:https://www.osapublishing.
org/viewmedia.cfm?URI=ol-44-15-3653&am=y>
DOI: 10.1364/OL.44.003653**
• **PARK JUNGHYUN ET AL: "All-solid-state spatial
light modulator with independent phase and
amplitude control for three-dimensional LiDAR
applications", NATURE NANOTECHNOLOGY,
vol. 16, no. 1, 26 October 2020 (2020-10-26),
pages 69 - 76, XP037334300, ISSN: 1748-3387,
DOI: 10.1038/S41565-020-00787-Y**
• **YAO-WEI HUANG ET AL: "Gate-Tunable
Conducting Oxide Metasurfaces", NANO
LETTERS,20200311AMERICAN CHEMICAL
SOCIETY, US, vol. 16, no. 9, 2 September 2016
(2016-09-02), pages 5319 - 5325, XP055462905,
ISSN: 1530-6984, DOI: 10.1021/
acs.nanolett.6b00555**

EP 4 561 305 B1

## Description

## BACKGROUND

Field of Disclosure

Some embodiments of the present disclosure relate to an integrated optical element and forming method of a metasurface.

Description of Related Art

Generally, a gate-tunable metasurface is a reflection-mode device, and a carrier concentration of the transparent conductive layer cladded in the structure is regulated by applying voltage to a gate. The device can provide different degree of phase shift at different location to regulate the deflection angle of the reflected light by changing the optical properties of the device. However, the antennas of the gate-tunable metasurface are usually at the wavelength location with lower reflectivity, and thus the device should be operated at the wavelength location with lower reflectivity, which cause low efficiency of the device. Zhang Jinqiannan ET AL ("Gate-tunable optical filter based on conducting oxide metasurface heterostructure", Optics Letters, vol. 44, no. 15, 18 July 2019 , page 3653, XP093248896, DOI: 10.1364/OL.44.003653) and Yao-Wei Huang ET AL ("Gate-Tunable Conducting Oxide Metasurfaces", Nano Letters, American Chemical Society, US, vol. 16, no. 9, 2 September 2016, pages 5319-5325, XP055462905, DOI: 10.1021/acs.nanolett.6b00555) each disclose such a prior art gate-tunable optical filter.

## SUMMARY

Some embodiments of the present disclosure provide an integrated optical element including a light-emitting device layer and a metasurface over the light-emitting device layer. The metasurface includes a plurality of conductive layers, a first dielectric layer and a first transparent conductive layer. The conductive layers are arranged along a first direction, in which each of the conductive layers has a plurality of holes. The first dielectric layer covers the conductive layers. The first transparent conductive layer covers the first dielectric layer.

In some embodiments, the metasurface further includes a substrate below the conductive layers.

In some embodiments, the first dielectric layer of the metasurface is in contact with the substrate.

In some embodiments, the metasurface further includes a second transparent conductive layer between the substrate and the conductive layers, and a second dielectric layer between the second transparent conductive layer and the conductive layers.

In some embodiments, the first dielectric layer of the metasurface is in contact with the second dielectric layer of the metasurface.

In some embodiments, the holes in each of the conductive layers are arranged in a second direction different from the first direction.

In some embodiments, the conductive layers are arranged in a two-dimensional array.

In some embodiments, the holes in each of the conductive layers comprise a plurality of first holes and a plurality of second holes, the first holes are arranged in a second direction different from the first direction, and one of the second holes is among the first holes, and one of the second holes is aligned with a center of a line connecting centers of two of the first holes, the two of the first holes are adjacent to each other.

In some embodiments, one of the holes is a concave quadrilateral.

In some embodiments, one of the holes comprises two circular holes connected by a rectangular hole.

Some embodiments of the present disclosure provide a method of forming a metasurface including forming a conductive material layer over the substrate, forming a plurality of conductive layers by patterning the conductive material layer, in which the conductive layers are arranged along a first direction, and each of the conductive layers has a plurality of holes, forming a first dielectric layer over the conductive layers, and forming a first transparent conductive layer over the first dielectric layer.

In some embodiments, the first dielectric layer extends from sidewalls of the conductive layers to an upper surface of the substrate when forming the first dielectric layer.

In some embodiments, before forming the conductive material layer, the method further includes forming a second transparent conductive layer over the substrate, and forming a second dielectric layer over the second transparent conductive layer, in which the second dielectric layer is between the second transparent conductive layer and the conductive layers.

In some embodiments, the first dielectric layer extends from sidewalls of the conductive layers to an upper surface of the second dielectric layer after forming the first dielectric layer over the conductive layers.

In some embodiments, the holes of each of the conductive layers are arranged along the first direction.

In some embodiments, the holes of each of the conductive layers are further arranged along a second direction different from the first direction.

In some embodiments, the conductive layers are arranged in a two-dimensional array.

In some embodiments, the holes in each of the conductive layers comprise a plurality of first holes and a plurality of second holes, the first holes are arranged in a second direction different from the first direction, and one of the second holes is among the first holes, and one of the second holes is aligned with a center of a line connecting centers of two of the first holes, the two of the first holes are adjacent to each other.

In some embodiments, one of the holes is a concave quadrilateral.

In some embodiments, one of the holes comprises two circular holes connected by a rectangular hole.

## BRIEF DESCRIPTION OF THE DRAWINGS

Fig. 1 illustrates a diagram of an integrated optical element in some embodiments of the present disclosure.

Fig. 2 illustrates a top view of a portion of the metasurface in Fig. 1.

Fig. 3 illustrates a cross-section view of the metasurface taken along the line A-A in Fig. 2 in some embodiments of the present disclosure.

Fig. 4 illustrates a cross-section view of the metasurface taken along the line A-A in Fig. 2 in some other embodiments of the present disclosure.

Figs. 5-8 illustrate cross-section views of a manufacturing process of the metasurface in Fig. 4 in some embodiments of the present disclosure.

Figs. 9 and 10 illustrate cross-section views of a manufacturing process of a metasurface in some other embodiments of the present disclosure.

Fig. 11 illustrates a top view of the metasurface in some other embodiments of the present disclosure.

Fig. 12 illustrates a top view of the metasurface in some other embodiments of the present disclosure.

Fig. 13 illustrates a top view of the metasurface in some other embodiments of the present disclosure.

Fig. 14 illustrates a top view of a protrusion structure in some embodiments of the present disclosure.

Fig. 15 illustrates a top view of a protrusion structure in some other embodiments of the present disclosure.

## DETAILED DESCRIPTION

Some embodiments of the present disclosure provide an optical element including a novel metasurface. The metasurface in some embodiments of the present disclosure are transmission-mode metasurfaces, and protrusions of the metasurface have holes. Therefore, the metasurface in some embodiments of the present disclosure are operated at wavelength location with higher transmittance to enhance the efficiency of the metasurface.

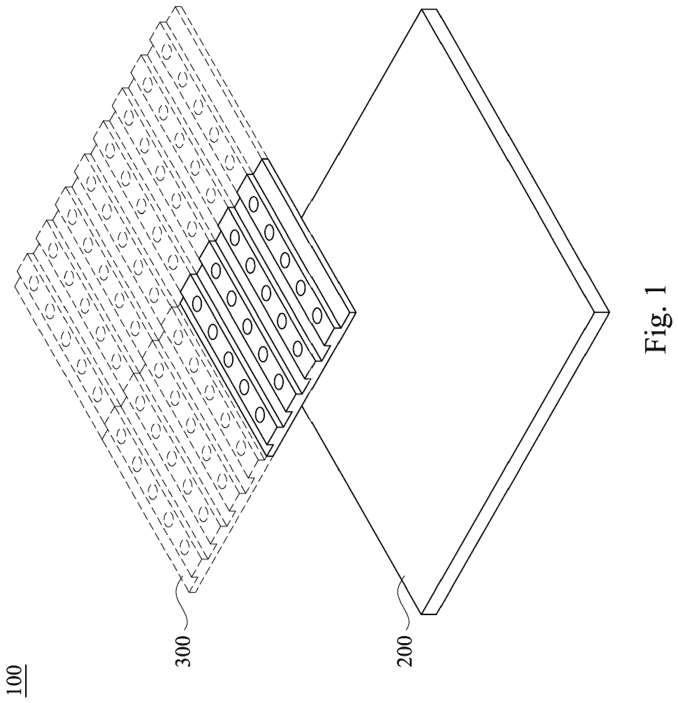

Fig. 1 illustrates a diagram of an integrated optical element 100 in some embodiments of the present disclosure. The integrated optical element 100 includes a light-emitting device layer 200 and a metasurface 300. The metasurface 300 is over the light-emitting device layer 200. The light-emitting device layer 200 is a layer including any forms of light-emitting device. For example, the light-emitting device layer 200 includes a carrier board and light-emitting devices arranged thereon. The light-emitting device may be light-emitting diode chip, micro light-emitting diode chip, organic light-emitting diode chip, semiconductor laser chip or the like.

The metasurface 300 is over the light-emitting device layer 200. The metasurface 300 in some embodiments of the present disclosure are transmission-mode metasurfaces. That is, the light emitting from the light source can penetrate the metasurface 300, and the metasurface 300 can change the optical properties of the light emitting from the light source. Therefore, the light-exiting direction of the metasurface 300 and the light-exiting direction of the light-emitting device layer 200 are the same.

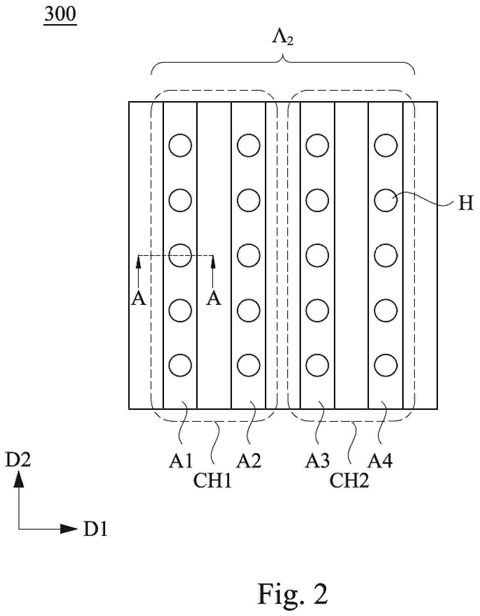

Fig. 2 illustrates a top view of a portion of the metasurface 300 in Fig. 1. The metasurface 300 includes many channels , such as channels CH1 and CH2 , and each of the channels includes many protrusion structures. For example, the channel CH1 may include protrusion structures A1 and A2, and the channel CH2 may include protrusion structures A3 and A4. According to the invention, the protrusion structures A1, A2, A3 and A4 include holes H, and protrusion structures A1, A2, A3 and A4 are arranged along a first direction D1 and extend along a second direction D2. According to the invention, each of the protrusion structure includes a row of holes H. Each hole H is an antenna unit. The size of the hole H can determine the wavelength range of light passing the metasurface 300. In some embodiments, holes H are sub-wavelength structures, and the diameters of the holes H are between 1/10 and 1/2 of the wavelength of the incident light.

The metasurface 300 may be used to change the deflection angle of the incident light. After the light enters the metasurface 300 from below, the deflection angle is represented by the following relational expression:

$$\theta = \sin^{-1}(\lambda/\Lambda).$$

$\theta$ is the deflection angle, $\lambda$ is the wavelength of the incident light, $\Lambda$ is the width of the period of the metasurface 300. The term "period" herein refers to the total width of specific numbers of the channels. For example, channels CH1 and CH2 in Fig. 2 may form a period $\Lambda_2$, and the channels CH1 and CH2 in the period $\Lambda_2$ may be used to regulate the same deflection angle. Therefore, specific periods are selected and corresponding voltage is applied based on specific deflection angles, and different channels in the period (such as the channels CH1 and CH2 in period $\Lambda_2$) are applied by different voltages. When applying voltage to the channels, the carrier concentration of the transparent conductive layer of the channel is changed, and the optical properties of the channel are also changed. When applying different voltages to different channels in the period, the carrier concentration of the transparent conductive layers of different channels are different (such as gradually changing along the first direction D1), and thus the optical properties of the different channel are different. As a result, the metasurface 300 may provide different degree of phase shift at different location, and the deflection angle of the incident light is regulated.

The numbers of the channels may determine the precision of the regulation ability of the light. For example, the more the channels in the period, the higher precision of the regulation ability of the light is achieved. In some embodiments, each period may include two channels, such as channels CH1 and channel CH2, and channel CH1 may include the protrusion structures A1 and A2, the channel CH2 may include the protrusion structure A3 and A4. However, the present disclosure is not limited thereto.

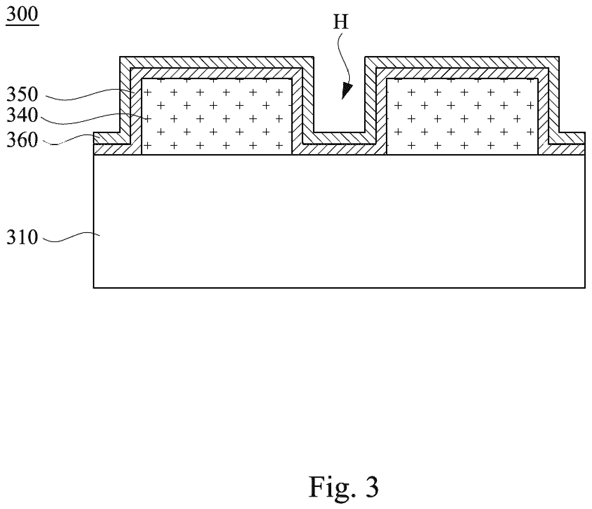

Fig. 3 illustrates a cross-section view of the metasurface 300 taken along the line A-A in Fig. 2 in some embodiments of the present disclosure. The metasurface 300 includes a substrate 310, a conductive layer 340, a dielectric layer 350 and a transparent conductive layer 360. Specifically, the conductive layer 340 is over the substrate 310 and in contact with the substrate 310. The conductive layer 340 has a specific shape, and the conductive layer 340 has the holes H. The conductive layer 340 determines the shapes of the protrusion structure A1, A2, A3 and A4 (Fig. 2). The dielectric layer 350 conformally covers the conductive layer 340, and the transparent conductive layer 360 conformally covers the dielectric layer 350. In some embodiments, the dielectric layer 350 further conformally covers the substrate 310, so the dielectric layer 350 extends along the upper surface of the substrate 310 to the sidewall and the upper surface of the conductive layer 340. That is, the conductive layer 340 is cladded by the dielectric layer 350 and the substrate 310. The conductive layer 340 and the transparent conductive layer 360 are made of different conductive material, and the conductive materials of the conductive layer 340 and the transparent conductive layer 360 have different carrier concentration. The carrier concentration of the conductive layer 340 is greater than the carrier concentration of the transparent conductive layer 360. For example, the carrier concentration of the conductive layer 340 is more than two orders of magnitude greater than the carrier concentration of the transparent conductive layer 360. In some embodiments, the order of magnitude of the carrier concentration of the conductive layer 340 is $10^{22}$/cm$^3$, and the order of magnitude of the carrier concentration of the transparent conductive layer 360 is $10^{20}$/cm$^3$. It is noted that Fig. 3 only illustrates the cross-section view of the protrusion structure A1. The cross-section views of other protrusion structures may be shown as Fig. 3, too. Therefore, the metasurface 300 may include a plurality of the conductive layers 340. The conductive layers 340 are over the substrate 310 and arranged along the first direction D1, and each of the conductive layers 340 includes the hole H, as shown in Fig. 2. Moreover, the transparent conductive layers 360 over each protrusion structure are not connected. Therefore, in subsequent operation, the transparent conductive layers 360 over each protrusion structure are independently regulated.

The metasurface 300 in Fig. 3 is a single-gate structure. That is, a voltage Vu may be applied to the transparent conductive layer 360, a voltage Vg (Vg is ground voltage generally) may be applied to the conductive layer 340, so the carrier concentration of a side of the transparent conductive layer 360 near the dielectric layer 350 is regulatable. The transparent conductive layers 360 of different channels are applied by different voltage Vu, so the carrier concentration of the transparent conductive layers in different channels are different (such as gradually changing along the first direction D1 in Fig. 2), and the optical properties are different among different channels. As a result, the metasurface 300 may provide different degree of phase shift at different location, and the deflection angle of the incident light is regulated. In this embodiment, since the channels are arranged along the first direction D1 (Fig. 2); that is, the channels form a one-dimensional metasurface, and the deflection angle of the incident light is regulated in one dimension by the metasurface 300.

In the present disclosure, the metasurface 300 is a transmission-mode metasurface, and the protrusion structures A1, A2, A3 and A4 of the metasurface 300 have the holes H. Compared to the reflection-mode metasurface, which is generally designed to be operated at the wavelength location with lower reflectivity and cause lower efficiency and tends to cause additional stray light and higher-order diffraction, the holes H of the metasurface 300 are negative structures designed based on Babinet's principle, and the plasma resonance mode caused by the holes H is at the wavelength with higher transmittance, which is beneficial for enhancing the efficiency of the metasurface 300. Moreover, the conductive layers 340 of the metasurface 300 have the holes H and the holes H are sub-wavelength structure, so the conductive layers 340 of the metasurface 300 may be used to avoid the higher-order diffraction (such as grating diffraction). As a result, the quality of penetrating light beam is enhanced. Moreover, since the metasurface 300 in the present disclosure is a transmission-mode metasurface, it is easy to combine the metasurface 300 and the light-emitting device layer to form the integrated optical element 100 (Fig. 1). If the metasurface is a reflection-mode metasurface, the light-emitting device layer is over the metasurface. If the light exits upwards from the integrated optical element, there are many design restrictions due to the existence of the light-emitting device layer.

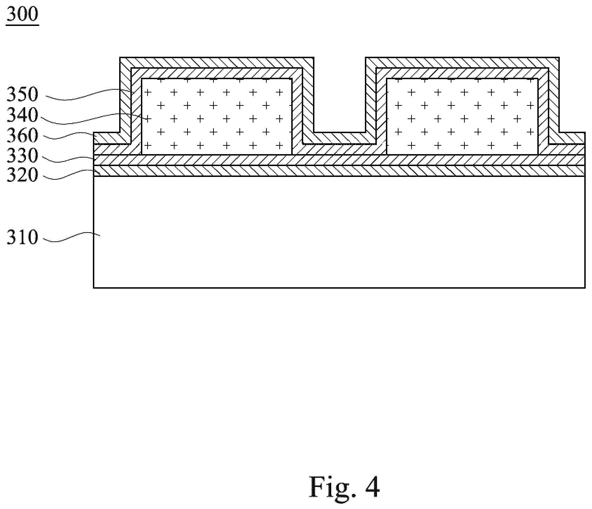

Fig. 4 illustrates a cross-section view of the metasurface 300 taken along the line A-A in Fig. 2 in some other embodiments of the present disclosure. The metasurface 300 in Fig. 4 is similar to the metasurface 300 in Fig. 3. The difference is that the metasurface 300 in Fig. 4 further includes the transparent conductive layer 320 and the dielectric layer 330. The transparent conductive layer 320 is between the substrate 310 and the conductive layer 340, the dielectric layer 330 is between the transparent conductive layer 320 and the conductive layer 340, and the conductive layer 340 is in contact with the dielectric layer 330 and is not in contact with the substrate 310. The dielectric layer 350 conformally covers the conductive layer 340, and the transparent conductive layer 360 conformally covers the dielectric layer 350. In some embodiments, dielectric layer 350 further conformally covers the dielectric layer 330, and the dielectric layer 350 extends along the upper surface of the dielectric layer 330 to the sidewalls and the upper surface of the conductive layer 340. The conductive layer 340 and the transparent conductive layers 320 and 360 are made of different conductive material, and the conductive materials of the conductive layer 340 and the transparent conductive layers 320 and 360 have different carrier concentration. The carrier concentration of the conductive layer 340 is greater than the carrier concentration of the transparent conductive layers 320 and 360. For example, the carrier concentration of the conductive layer 340 is more than two orders of magnitude greater than the carrier concentration of the transparent conductive layers 320 and 360. In some embodiments, the order of magnitude of the carrier concentration of the conductive layer 340 is $10^{22}/cm^3$, and the order of magnitude of the carrier concentration of the transparent conductive layers 320 and 360 is $10^{20}/cm^3$. It is noted that Fig. 4 only illustrates the cross-section view of the protrusion structure A1. The cross-section views of other protrusion structures may be shown as Fig. 4, too. Therefore, the metasurface 300 may include a plurality of the conductive layers 340. The conductive layers 340 are over the substrate 310 and arranged along the first direction D1, and each of the conductive layers 340 includes the hole H, as shown in Fig. 2. Moreover, the transparent conductive layers 360 over each protrusion structure are not connected, and the transparent conductive layers 320 below each protrusion structure are not connected. Therefore, in subsequent operation, the transparent conductive layers 360 over each protrusion structure and the transparent conductive layers 320 below each protrusion structure are independently regulated.

The metasurface 300 in Fig. 4 is a dual-gate structure. That is, a voltage Vu and a voltage Vb may be applied to the transparent conductive layer 360 and the transparent conductive layer 320 respectively, a voltage Vg (Vg is ground voltage generally) may be applied to the conductive layer 340, so the carrier concentration of a side of the transparent conductive layer 360 near the dielectric layer 350 and the carrier concentration of a side of the transparent conductive layer 320 near the dielectric layer 350 is regulatable. The transparent conductive layers 320 of different channels are applied by different voltage Vb, the transparent conductive layers 360 of different channels are applied by different voltage Vu, so the carrier concentration of the transparent conductive layers in different channels are different (such as gradually changing along the first direction D1 in Fig. 2), and the optical properties are different among different channels. As a result, the metasurface 300 may provide different degree of phase shift at different location, and the deflection angle of the incident light is regulated. In some embodiments, the dual-gate structure of the metasurface 300 may provide better ability of light beam regulation than the single-gate structure of the metasurface 300. In this embodiment, since the channels are arranged along the first direction D1 (Fig. 2); that is, the channels form a one-dimensional metasurface, and the deflection angle of the incident light is regulated in one dimension by the metasurface 300.

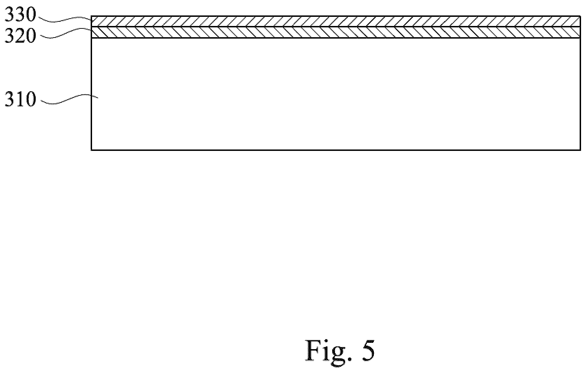

Figs. 5-8 illustrate cross-section views of a manufacturing process of the metasurface 300 in Fig. 4 in some embodiments of the present disclosure. Referring to Fig. 5, a substrate 310 is provided. A transparent conductive layer 320 is formed over the substrate 310, and then a dielectric layer 330 is formed over the transparent conductive layer 320. The transparent conductive layer 320 and the dielectric layer 330 are planar layer over the substrate 310. In some embodiments, the substrate 310 may be glass substrate or other suitable transparent substrate, so that the light emitting from the light source below can penetrate the metasurface 300. In some embodiments, the transparent conductive layer 320 may be made of indium tin oxide (ITO) or other suitable transparent conductive material. The dielectric layer 330 may be made of high-k dielectric material, such as $Al_2O_3$ or $HfO_2$. In some embodiments, the dielectric layer 330 may be formed by atomic layer deposition.

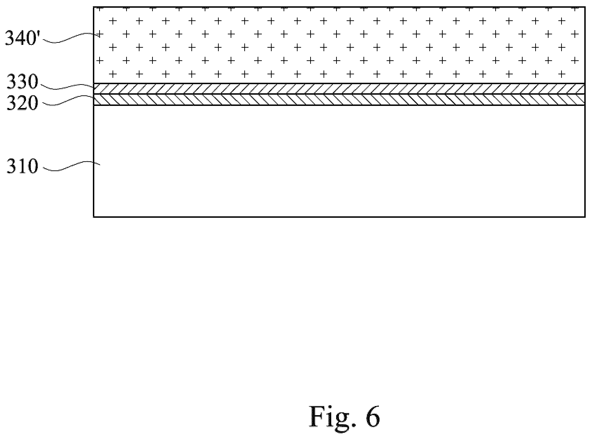

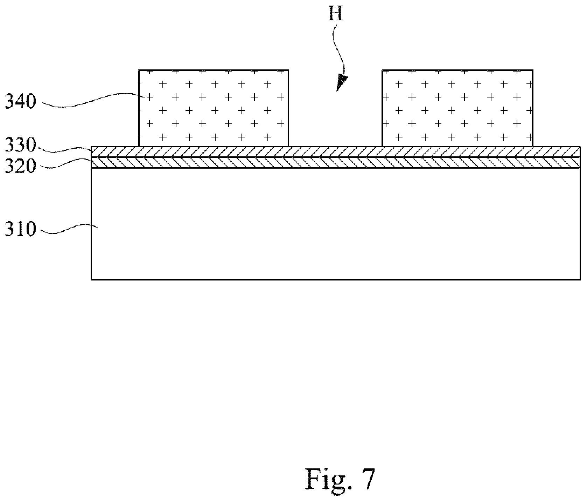

Referring to Fig. 6, a conductive material layer 340' is formed over the dielectric layer 330. Subsequently, referring to Fig. 7, conductive layers 340 are formed by patterning the conductive material layer 340', the conductive layers 340 are arranged along the first direction (such as first direction D1 in Fig. 2) and the conductive layers 340 have a plurality of holes H. Specifically, the patterned conductive layers 340 form a plurality of protrusion structures (such as protrusion structures A1, A2, A3 and A4 in Fig.2), and each protrusion structure includes a plurality of holes H. The shape of the conductive layer 340 and the diameter of the holes H are determined based on actual situation. In some embodiments, the material of the conductive layer 340 may be selected based on applicable wavelength. For example, when the metasurface 300 is applicable for infrared light, the conductive layer 340 may be made of gold. When the metasurface 300 is applicable for ultra violet, the conductive layer 340 may be made of aluminum. When the metasurface 300 is applicable for blue light, the conductive layer 340 may be made of silver.

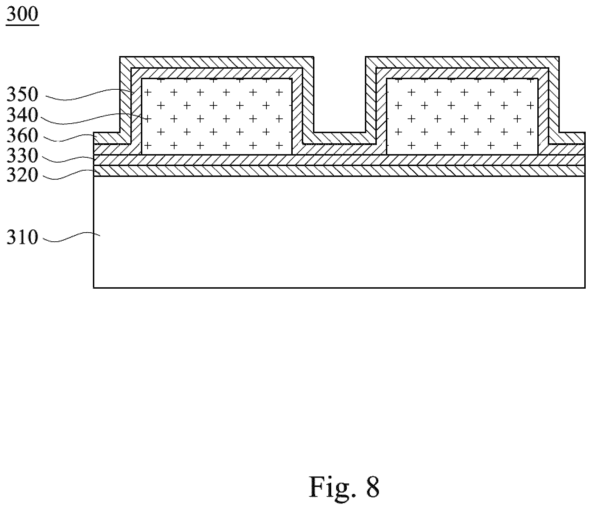

Referring to Fig. 8, a dielectric layer 350 is formed over the conductive layer 340. After forming the dielectric layer 350 over the conductive layer 340, the dielectric layer 350 extends from the sidewall of the conductive layer 340 to the upper surface of the dielectric layer 330. The conductive layer 340 is cladded and surrounded by the dielectric layer 350 and the dielectric layer 330. Subsequently, a transparent conductive layer 360 is formed over the dielectric layer 350. In some embodiments, the dielectric layer 350 may be made of high-k dielectric material, such as $Al_2O_3$ or $HfO_2$. In some embodiments, the dielectric layer 350 may be formed by atomic layer deposition. The transparent conductive layer 360 may be made of indium tin oxide (ITO) or other suitable transparent conductive material. After forming the metasurface 300, the metasurface 300 may be disposed over the light-emitting device layer 200. Therefore, the metasurface 300 may be used to regulate the physical properties of the light emitting from the light-emitting device layer 200.

The manufacturing process of the metasurface 300 in Fig. 3 is similar to the processes in Figs. 5-8. The difference is that the transparent conductive layer 320 and the dielectric layer 330 in Fig. 5 are omitted when forming the metasurface 300 in Fig. 3. Therefore, in Fig. 6, the conductive material layer 340' may be directly formed over the substrate 310, and the subsequent processes are continued. When forming the metasurface 300 in Fig.3, after forming the dielectric layer 350 over the conductive layer 340, the dielectric layer 350 extends from the sidewalls of the conductive layer 340 to the upper surface of the substrate 310. The conductive layer 340 is cladded by the dielectric layer 350 and the substrate 310.

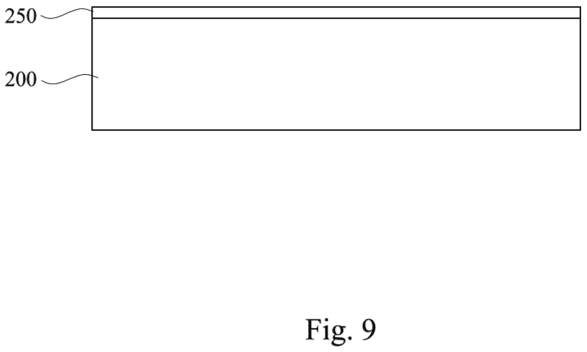

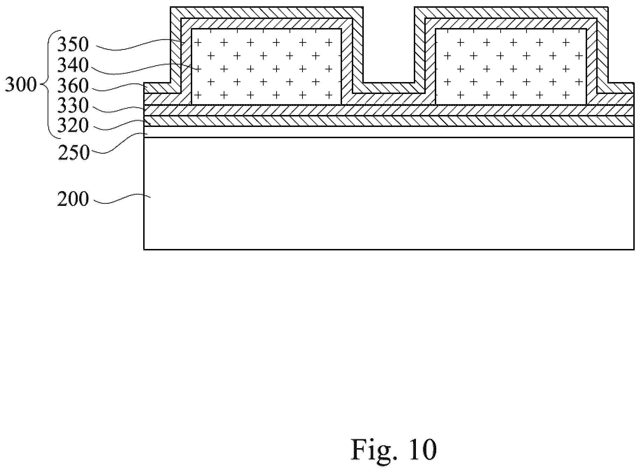

Figs. 9 and 10 illustrate cross-section views of a manufacturing process of a metasurface 300 in some other embodiments of the present disclosure. In Figs. 9 and 10, the metasurface 300 is directly formed over the light-emitting device layer 200. Specifically, referring to Fig. 9, a dielectric layer 250 is formed over the light-emitting device layer 200. The dielectric layer 250 may be made of silicon oxide, silicon nitride, or the like.

Referring to Fig. 10, the metasurface 300 is formed over the dielectric layer 250. The forming method of the metasurface 300 is similar to the processes illustrated in Figs. 5-8. The difference is that the transparent conductive layer 320 is formed over the dielectric layer 250 in Fig. 10. As a result, the metasurface 300 may be directly formed over the light-emitting device layer 200.

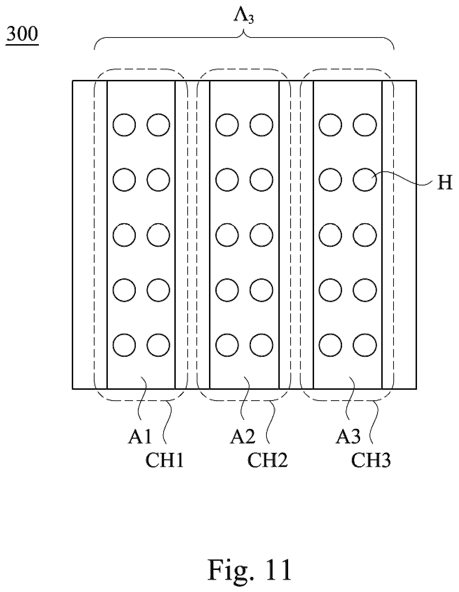

Fig. 11 illustrates a top view of the metasurface 300 in some other embodiments of the present disclosure. The metasurface 300 in Fig. 11 is similar to the metasurface 300 in Fig. 2. The difference is that the number of the channels in the metasurface 300 in Fig. 11 is different from the number of the channels in the metasurface 300 in Fig. 2, and the number of the holes H of each protrusion structure in the metasurface 300 in Fig. 11 is different from the number of the holes H of each protrusion structure in the metasurface 300 in Fig. 2. For example, channels CH1, CH2 and CH3 in Fig. 11 may form a period $\Lambda_3$. In this embodiment, the channels CH1, CH2 and CH3 are applied by different voltages respectively, and thus the optical properties of the different channels are different. As a result, the metasurface 300 may provide different degree of phase shift at different location, and the deflection angle of the incident light is regulated. When the number of the channels in each period increases, the deflection angle of the incident light is much precisely regulated. Moreover, each protrusion structure of the metasurface 300 in Fig. 11 includes two rows of the holes H. The number of the holes H in each protrusion structure is designed based on actual situation.

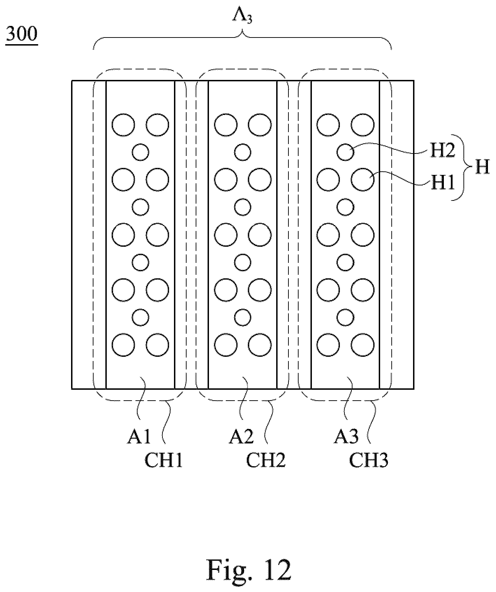

Fig. 12 illustrates a top view of the metasurface 300 in some other embodiments of the present disclosure. The metasurface 300 in Fig. 12 is similar to the metasurface 300 in Fig. 11. The difference is that the holes H in Fig. 12 include first holes H1 and second holes H2, the first holes H1 are arranged along the first direction D1 and the second direction D2. The second hole H2 is arranged among four first holes H1, the second hole H2 is aligned with a center of a line connecting centers of two adjacent first holes H1. Therefore, the number of the holes H increases per unit area, and the ability of the light beam regulation of the metasurface 300 may be enhanced.

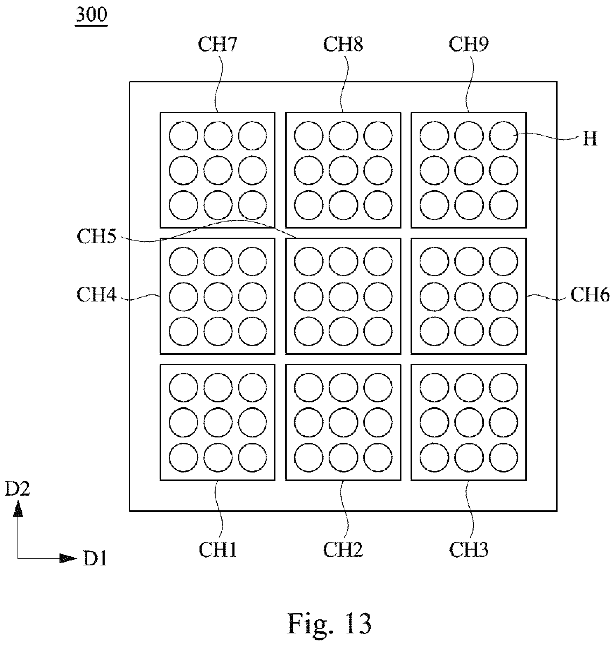

Fig. 13 illustrates a top view of the metasurface 300 in some other embodiments of the present disclosure. The metasurface 300 in Fig. 13 includes channels CH1, CH2, CH3, CH4, CH5, CH6, CH7, CH8 and CH9, and each channel may include one protrusion structure. The protrusion structures are arranged along the first direction D1 and further arranged along the second direction D2, and the first direction D1 is perpendicular to the second direction D2. Therefore, the channels (i.e. protrusion structures) of the metasurface 300 in Fig. 13 are arranged in a two-dimensional array. As a result, the channels CH1, CH2, CH3, CH4, CH5, CH6, CH7, CH8 and CH9 in Fig. 13 are applied by different voltages respectively, and thus the optical properties of the different channels are different. As a result, the metasurface 300 may provide different degree of phase shift at different location, and the deflection angle of the incident light is regulated. When the channels form a two-dimensional metasurface, the deflection angle of the incident light is regulated in two dimensions by the metasurface 300.

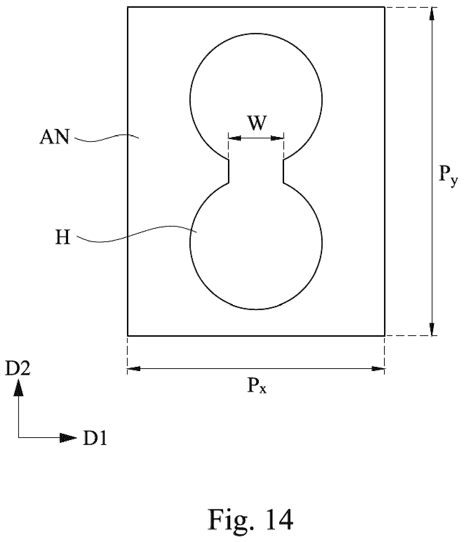

Fig. 14 illustrates a top view of a protrusion structure AN in some embodiments of the present disclosure. The protrusion structure AN in Fig. 14 may have a hole H, and the hole H is in a dumbbell shape. Specifically, the hole H is two circular holes connected by a rectangular hole. The protrusion structure AN is rectangular, and the length Px of the protrusion structure AN in the first direction D1 is 400 nm, the length Py of the protrusion structure AN in the second direction D2 is 600 nm, and the width W of the dumbbell neck of the hole H is 100 nm. In the case where the protrusion structure AN has the shape mentioned above, the protrusion structure AN is operated at the wavelength location with higher transmittance. For example, when the deflection angle of the incident light is regulated by using the protrusion structure AN in Fig. 14, the wavelength is about 1.5 micron, the transmittance of the incident light can reach 92%, and the ability of phase shift regulation can reach 141.6 degrees. The protrusion structures AN may be arranged in a one-dimensional array or a two-dimensional array. Therefore, the deflection angle of the incident light is regulated in one dimension or two dimensions by the metasurface including the protrusion structures AN.

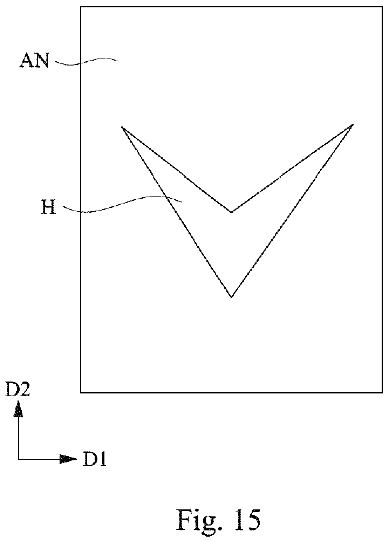

Fig. 15 illustrates a top view of a protrusion structure AN in some other embodiments of the present disclosure. The protrusion structure AN in Fig. 15 may have a hole H, and the hole H is in a boomerang shape. Specifically, the hole H may be a concave quadrilateral. In some embodiments, four corners of the concave quadrilateral are formed by straight lines, as shown in Fig. 15. In some other embodiments, four corners of the concave quadrilateral are round corners.

As mentioned above, the transmission-mode metasurface in some embodiments of the present disclosure has many advantages. The metasurface has holes designed based on Babinet's principle, and the plasma resonance mode caused by the holes H is at the wavelength with higher transmittance, which is beneficial for enhancing the efficiency of the metasurface. Moreover, the conductive layers of the metasurface have the holes and the holes are sub-wavelength structure, so the conductive layers of the metasurface may be used to avoid the higher-order diffraction (such as grating diffraction). As a result, the quality of penetrating light beam is enhanced. Moreover, since the metasurface in the present disclosure is a transmission-mode metasurface, it is easy to combine the metasurface and the light-emitting device layer to form the integrated optical element. The light-emitting device layer will not interfere the light-exiting direction of the integrated optical element.

**Claims**

1. An integrated optical element (100), comprising:

    a light-emitting device layer (200); and
    a metasurface (300) over the light-emitting device layer (200), wherein the metasurface (300) comprises:

        a plurality of conductive layers (340) arranged along a first direction (D1), wherein each of the conductive layers (340) has a plurality of holes (H);
        a first dielectric layer (350) covering the conductive layers (340); and
        a first transparent conductive layer (360) covering the first dielectric layer (350).

2. The integrated optical element (100) of claim 1, wherein the metasurface (300) further comprises a substrate (310) below the conductive layers (340).

3. The integrated optical element (100) of claim 2, wherein the first dielectric layer (350) of the metasurface (300) is in contact with the substrate (310).

4. The integrated optical element (100) of claim 1 or 2, wherein the metasurface (300) further comprises:

    a second transparent conductive layer (320) between the substrate (310) and the conductive layers (340); and
    a second dielectric layer (330) between the second transparent conductive layer (320) and the conductive layers (340).

5. The integrated optical element (100) of claim 4, wherein the first dielectric layer (350) of the metasurface (300) is in contact with the second dielectric layer (330) of the metasurface (300).

6. The integrated optical element (100) of claim 1, 2, 3, 4 or 5, wherein the holes (H) in each of the conductive layers (340) are arranged in a second direction (D2) different from the first direction (D1).

7. The integrated optical element (100) of claim 1, 2, 3, 4 or 5, wherein the conductive layers (340) are arranged in a two-dimensional array.

8. The integrated optical element (100) of claim 1, 2, 3, 4 or 5, wherein the holes (H) in each of the conductive layers (340) comprise a plurality of first holes (H1) and a plurality of second holes (H2), the first holes (H1) are arranged in a second direction (D2) different from the first direction (D1), and one of the second holes (D2) is among the first holes (D1), and one of the second holes (D2) is aligned with a center of a line connecting centers of two of the first holes (D1), the two of the first holes (D1) are adjacent to each other.

9. The integrated optical element (100) of claim 1, 2, 3, 4, 5, 6, 7, or 8, wherein one of the holes (H) is a concave quadrilateral.

10. The integrated optical element (100) of claim 1, 2, 3, 4, 5, 6, 7, or 8, wherein one of the holes (H) comprises two circular holes connected by a rectangular hole.

11. A method of forming a metasurface (300), comprising:

    forming a conductive material layer (340') over a substrate (300);
    forming a plurality of conductive layers (340) by patterning the conductive material layer (340'), wherein the conductive layers (340) are arranged along a first direction (D1), and each of the conductive layers (340) has a plurality of holes (H);

forming a first dielectric layer (350) over the conductive layers (340); and

forming a first transparent conductive layer (360) over the first dielectric layer (350).

12. The method of claim 11, wherein the first dielectric layer (350) extends from sidewalls of the conductive layers (340) to an upper surface of the substrate (310) when forming the first dielectric layer (350).

13. The method of claim 11, wherein before forming the conductive material layer (340), the method further comprises:

forming a second transparent conductive layer (320) over the substrate (310); and

forming a second dielectric layer (330) over the second transparent conductive layer (320), wherein the second dielectric layer (330) is between the second transparent conductive layer (320) and the conductive layers (340).

14. The method of claim 11 or 13, the first dielectric layer (350) extends from sidewalls of the conductive layers (340) to an upper surface of the second dielectric layer (330) after forming the first dielectric layer (350) over the conductive layers (340).

15. The method of claim 11, 12, 13 or 14, wherein the holes (H) of each of the conductive layers (340) are arranged along the first direction (D1).

16. The method of claim 11, 12, 13 or 14, wherein the holes of (H) each of the conductive layers (340) are further arranged along a second direction (D2) different from the first direction (D1).

17. The method of claim 11, 12, 13 or 14, wherein the conductive layers (340) are arranged in a two-dimensional array.

18. The method of claim 11, 12, 13 or 14, wherein the holes (H) in each of the conductive layers (340) comprise a plurality of first holes (H1) and a plurality of second holes (H2), the first holes (H1) are arranged in a second direction (D2) different from the first direction (D1), and one of the second holes (H2) is among the first holes (H1), and one of the second holes (H2) is aligned with a center of a line connecting centers of two of the first holes (H1), the two of the first holes (H1) are adjacent to each other.

19. The method of claim 11, 12, 13 or 14, wherein one of the holes (H) is a concave quadrilateral.

20. The method of claim 11, 12, 13 or 14, wherein one of the holes (H) comprises two circular holes connected by a rectangular hole.

**Patentansprüche**

1. Integriertes optisches Element (100) mit:

einer lichtemittierenden Vorrichtungsschicht (200); und

einer Metafläche (300) über der lichtemittierenden Vorrichtungsschicht (200),

wobei die Metafläche (300) Folgendes aufweist:

eine Vielzahl von leitfähigen Schichten (340), die entlang einer ersten Richtung (D1) angeordnet sind, wobei jede der leitfähigen Schichten (340) eine Vielzahl von Löchern (H) aufweist;

eine erste dielektrische Schicht (350), die die leitfähigen Schichten (340) bedeckt; und

eine erste transparente leitfähige Schicht (360), die die erste dielektrische Schicht (350) bedeckt.

2. Integriertes optisches Element (100) nach Anspruch 1, wobei die Metafläche (300) ferner ein Substrat (310) unter den leitfähigen Schichten (340) aufweist.

3. Integriertes optisches Element (100) nach Anspruch 2, wobei die erste dielektrische Schicht (350) der Metafläche (300) in Kontakt mit dem Substrat (310) ist.

4. Integriertes optisches Element (100) nach Anspruch 1 oder 2, wobei die Metafläche (300) ferner Folgendes aufweist:

eine zweite transparente leitfähige Schicht (320) zwischen dem Substrat (310) und den leitfähigen Schichten (340); und

eine zweite dielektrische Schicht (330) zwischen der zweiten transparenten leitfähigen Schicht (320) und den leitfähigen Schichten (340).

5. Integriertes optisches Element (100) nach Anspruch 4, wobei die erste dielektrische Schicht (350) der Metafläche (300) in Kontakt mit der zweiten dielektrischen Schicht (330) der Metafläche (300) ist.

6. Integriertes optisches Element (100) nach Anspruch 1, 2, 3, 4 oder 5, wobei die Löcher (H) in jeder der leitfähigen Schichten (340) in einer zweiten Richtung (D2) angeordnet sind, die sich von der ersten Richtung (D1) unterscheidet.

7. Integriertes optisches Element (100) nach Anspruch 1, 2, 3, 4 oder 5, wobei die leitfähigen Schichten (340) in einer zweidimensionalen Anordnung angeordnet sind.

8. Integriertes optisches Element (100) nach Anspruch 1, 2, 3, 4 oder 5, wobei die Löcher (H) in jeder der leitfähigen Schichten (340) eine Vielzahl von ersten Löchern (H1) und eine Vielzahl von zweiten Löchern (H2) aufweisen, wobei die ersten Löcher (H1) in einer zweiten Richtung (D2) angeordnet sind, die sich von der ersten Richtung (D1) unterscheidet, und eines der zweiten Löcher (D2) zwischen den ersten Löchern (D1) ist und eines der zweiten Löcher (D2) mit einer Mitte einer Linie ausgerichtet ist, die die Mitten von zwei der ersten Löcher (D1) verbindet, wobei die zwei der ersten Löcher (D1) benachbart zueinander sind.

9. Integriertes optisches Element (100) nach Anspruch 1, 2, 3, 4, 5, 6, 7 oder 8, wobei eines der Löcher (H) ein konkaves Viereck ist.

10. Integriertes optisches Element (100) nach Anspruch 1, 2, 3, 4, 5, 6, 7 oder 8, wobei eines der Löcher (H) zwei kreisförmige Löcher aufweist, die durch ein rechteckiges Loch verbunden sind.

11. Verfahren zum Bilden einer Metafläche (300), das Folgendes aufweist:

    Bilden einer leitfähigen Materialschicht (340') über einem Substrat (300);
    Bilden einer Vielzahl von leitfähigen Schichten (340) durch Strukturieren der leitfähigen Materialschicht (340'), wobei die leitfähigen Schichten (340) entlang einer ersten Richtung (D1) angeordnet sind und jede der leitfähigen Schichten (340) eine Vielzahl von Löchern (H) aufweist;
    Bilden einer ersten dielektrischen Schicht (350) über den leitfähigen Schichten (340); und
    Bilden einer ersten transparenten leitfähigen Schicht (360) über der ersten dielektrischen Schicht (350).

12. Verfahren nach Anspruch 11, wobei sich die erste dielektrische Schicht (350) von Seitenwänden der leitfähigen Schichten (340) zu einer oberen Fläche des Substrats (310) erstreckt, wenn die erste dielektrische Schicht (350) gebildet wird.

13. Verfahren nach Anspruch 11, wobei das Verfahren vor dem Bilden der leitfähigen Materialschicht (340) ferner Folgendes aufweist:

    Bilden einer zweiten transparenten leitfähigen Schicht (320) über dem Substrat (310); und
    Bilden einer zweiten dielektrischen Schicht (330) über der zweiten transparenten leitfähigen Schicht (320), wobei sich die zweite dielektrische Schicht (330) zwischen der zweiten transparenten leitfähigen Schicht (320) und den leitfähigen Schichten (340) befindet.

14. Verfahren nach Anspruch 11 oder 13, wobei sich die erste dielektrische Schicht (350) von Seitenwänden der leitfähigen Schichten (340) zu einer oberen Fläche der zweiten dielektrischen Schicht (330) erstreckt, nachdem die erste dielektrische Schicht (350) über den leitfähigen Schichten (340) gebildet wurde.

15. Verfahren nach Anspruch 11, 12, 13 oder 14, wobei die Löcher (H) jeder der leitfähigen Schichten (340) entlang der ersten Richtung (D1) angeordnet sind.

16. Verfahren nach Anspruch 11, 12, 13 oder 14, wobei die Löcher (H) jeder der leitfähigen Schichten (340) ferner entlang einer zweiten Richtung (D2) angeordnet sind, die sich von der ersten Richtung (D1) unterscheidet.

17. Verfahren nach Anspruch 11, 12, 13 oder 14, wobei die leitfähigen Schichten (340) in einer zweidimensionalen Anordnung angeordnet sind.

18. Verfahren nach Anspruch 11, 12, 13 oder 14, wobei die Löcher (H) in jeder der leitfähigen Schichten (340) eine Vielzahl von ersten Löchern (H1) und eine Vielzahl von zweiten Löchern (H2) aufweisen, wobei die ersten Löcher (H1) in einer zweiten Richtung (D2) angeordnet sind, die sich von der ersten Richtung (D1) unterscheidet, und eines der zweiten Löcher (H2) zwischen den ersten Löchern (H1) ist und eines der zweiten Löcher (H2) mit einer Mitte einer Linie ausgerichtet ist, die die Mitten von zwei der ersten Löcher (H1) verbindet, wobei die zwei der ersten Löcher (H1) benachbart zueinander sind.

19. Verfahren nach Anspruch 11, 12, 13 oder 14, wobei eines der Löcher (H) ein konkaves Viereck ist.

20. Verfahren nach Anspruch 11, 12, 13 oder 14, wobei eines der Löcher (H) zwei kreisförmige Löcher aufweist, die durch ein rechteckiges Loch verbunden sind.

**Revendications**

1. Élément optique intégré (100), comprenant :

    une couche de dispositif électroluminescent (200) ; et
    une métasurface (300) sur la couche de dispositif électroluminescent (200), dans laquelle la métasurface (300) comprend :

        une pluralité de couches conductrices (340) agencées selon une première direction (D1), dans lesquelles chacune des couches conductrices (340) présente une pluralité de trous (H) ;

une première couche diélectrique (350) recouvrant les couches conductrices (340) ; et une première couche conductrice transparente (360) recouvrant la première couche diélectrique (350).

2. Élément optique intégré (100) selon la revendication 1, dans lequel la métasurface (300) comprend en outre un substrat (310) situé sous les couches conductrices (340).

3. Élément optique intégré (100) selon la revendication 2, dans lequel la première couche diélectrique (350) de la métasurface (300) est en contact avec le substrat (310).

4. Élément optique intégré (100) selon la revendication 1 ou 2, dans lequel la métasurface (300) comprend en outre :

une seconde couche conductrice transparente (320) comprise entre le substrat (310) et les couches conductrices (340) ; et une seconde couche diélectrique (330) comprise entre la seconde couche conductrice transparente (320) et les couches conductrices (340).

5. Élément optique intégré (100) selon la revendication 4, dans lequel la première couche diélectrique (350) de la métasurface (300) est en contact avec la seconde couche diélectrique (330) de la métasurface (300).

6. Élément optique intégré (100) selon la revendication 1, 2, 3, 4 ou 5, dans lequel les trous (H) dans chacune des couches conductrices (340) sont agencés suivant une seconde direction (D2) différente de la première direction (D1).

7. Élément optique intégré (100) selon la revendication 1, 2, 3, 4 ou 5, dans lequel les couches conductrices (340) sont agencées dans un réseau bidimensionnel.

8. Élément optique intégré (100) selon la revendication 1, 2, 3, 4 ou 5, dans lequel les trous (H) dans chacune des couches conductrices (340) comprennent une pluralité de premiers trous (H1) et une pluralité de seconds trous (H2), les premiers trous (H1) sont agencés suivant une seconde direction (D2) différente de la première direction (D1), et l'un des seconds trous (D2) est parmi les premiers trous (D1), et l'un des seconds trous (D2) est aligné avec le centre d'une ligne reliant les centres de deux des premiers trous (D1), les deux premiers trous (D1) sont adjacents l'un à l'autre.

9. Élément optique intégré (100) selon la revendication 1, 2, 3, 4, 5, 6, 7 ou 8, dans lequel l'un des trous (H) est un quadrilatère concave.

10. Élément optique intégré (100) selon la revendication 1, 2, 3, 4, 5, 6, 7 ou 8, dans lequel l'un des trous (H) comprend deux trous circulaires reliés par un trou rectangulaire.

11. Procédé de formation d'une métasurface (300), comprenant :

la formation d'une couche de matériau conducteur (340') sur un substrat (300) ; la formation d'une pluralité de couches conductrices (340) en structurant la couche de matériau conducteur (340'), dans laquelle les couches conductrices (340) sont agencées selon une première direction (D1), et chacune des couches conductrices (340) présente une pluralité de trous (H) ; la formation d'une première couche diélectrique (350) sur les couches conductrices (340) ; et la formation d'une première couche conductrice transparente (360) sur la première couche diélectrique (350).

12. Procédé selon la revendication 11, dans lequel la première couche diélectrique (350) s'étend des parois latérales des couches conductrices (340) à une surface supérieure du substrat (310) lors de la formation de la première couche diélectrique (350).

13. Procédé selon la revendication 11, dans lequel, avant de former la couche de matériau conducteur (340), le procédé comprend en outre :

la formation d'une seconde couche conductrice transparente (320) sur le substrat (310) ; et la formation d'une seconde couche diélectrique (330) sur la seconde couche conductrice transparente (320), dans laquelle la seconde couche diélectrique (330) est comprise entre la seconde couche conductrice transparente (320) et les couches conductrices (340).

14. Procédé selon la revendication 11 ou 13, la première couche diélectrique (350) s'étend des parois latérales des couches conductrices (340) à une surface supérieure de la seconde couche diélectrique (330) après la formation de la première couche diélectrique (350) sur les couches conductrices (340).

15. Procédé selon la revendication 11, 12, 13 ou 14, dans lequel les trous (H) de chacune des couches conductrices (340) sont agencés selon la première direction (D1).

16. Procédé selon la revendication 11, 12, 13 ou 14, dans lequel les trous (H) de chacune des couches conductrices (340) sont agencés en outre selon une seconde direction (D2) différente de la première direction (D1).

17. Procédé selon la revendication 11, 12, 13 ou 14, dans lequel les couches conductrices (340) sont agencées dans un réseau bidimensionnel.

18. Procédé selon la revendication 11, 12, 13 ou 14, dans lequel les trous (H) de chacune des couches conductrices (340) comprennent une pluralité de premiers trous (H1) et une pluralité de seconds trous (H2), les premiers trous (H1) sont agencés suivant une seconde direction (D2) différente de la première direction (D1), et l'un des seconds trous (H2) est parmi les premiers trous (H1), et l'un des seconds trous (H2) est aligné avec le centre d'une ligne reliant les centres de deux des premiers trous (H1), les deux premiers trous (H1) sont adjacents l'un à l'autre.

19. Procédé selon la revendication 11, 12, 13 ou 14, dans lequel l'un des trous (H) est un quadrilatère concave.

20. Procédé selon la revendication 11, 12, 13 ou 14, dans lequel l'un des trous (H) comprend deux trous circulaires reliés par un trou rectangulaire.

Fig. 1

Fig. 2

Fig. 3

Fig. 4

300

350
340
360
330
320

310

Fig. 5

Fig. 6

EP 4 561 305 B1

H

340

330

320

310

Fig. 7

350

340

360

330

320

310

EP 4 561 305 B1

Fig. 8

Fig. 9

250
200

Fig. 10

EP 4 561 305 B1

Fig. 11

Fig. 12

Fig. 13

Fig. 14

Fig. 15

AN

H

D2

D1

EP 4 561 305 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ZHANG JINQIANNAN et al.** Gate-tunable optical filter based on conducting oxide metasurface hetero-structure. *Optics Letters*, 18 July 2019, vol. 44 (15), 3653

- Gate-Tunable Conducting Oxide Metasurfaces. **YAO-WEI HUANG et al.** Nano Letters. American Chemical Society, 02 September 2016, vol. 16, 5319-5325